# EUROPEAN PATENT APPLICATION

(11) **EP 2 351 510 A1**
(43) Date of publication of application: **03.08.2011**
(21) Application number: 09824724.0
(22) Date of filing: 27.10.2009
(51) Int. Cl.: A61B 1/00

(54) **RADIO DEVICE FOR ACQUIRING IN-EXAMINEE INFORMATION**

(30) Priority: 05.11.2008 JP 2008284715
(71) Applicant: Olympus Corporation, Shibuya-ku Tokyo 151-0072 (JP)
(72) Inventor: SATO Ken, Tokyo 151-0072 (JP); IWAISAKO, Hiroshi, Tokyo 151-0072 (JP); MIYAHARA, Hideharu, Tokyo 151-0072 (JP)
(74) Representative: Winter, Brandl, Fürniss, Hübner, Röss, Kaiser, Polte - Partnerschaft
(86) International application number: PCT/JP2009/068422
(87) International publication number: WO 2010/053024

(57) **Abstract**

Provided is a wireless-type subject in-vivo information acquiring apparatus that is introduced into a body cavity of an individual to be examined and acquires subject in-vivo information, and that includes a transmission antenna that has an antenna orientation direction in which a transmission signal that transmits acquired subject in-vivo information is emitted most strongly, and another antenna that has an antenna directivity in a predetermined direction that is different from that of the transmission antenna. The transmission antenna and the other antenna are arranged so that a relationship between the antenna orientation direction of the transmission antenna and the antenna orientation direction of the other antenna is a twisted positional relationship or an intersecting relationship.

## Description

### Technical Field

The present invention relates to a wireless-type subject in-vivo information acquiring apparatus that is introduced into a body cavity of an individual to be examined and acquires subject in-vivo information.

### Background Art

A capsule-type medical device has come into practical use as a wireless-type subject in-vivo information acquiring apparatus that is introduced into a body cavity of an individual to be examined by being swallowed from the oral cavity.

When using such kind of capsule-type medical device, a method is sometimes adopted in which in-vivo information regarding a body cavity of the individual to be examined that is acquired by the capsule-type medical device is wirelessly transmitted by a transmission antenna to a signal receiving apparatus that is outside the body of the individual to be examined.

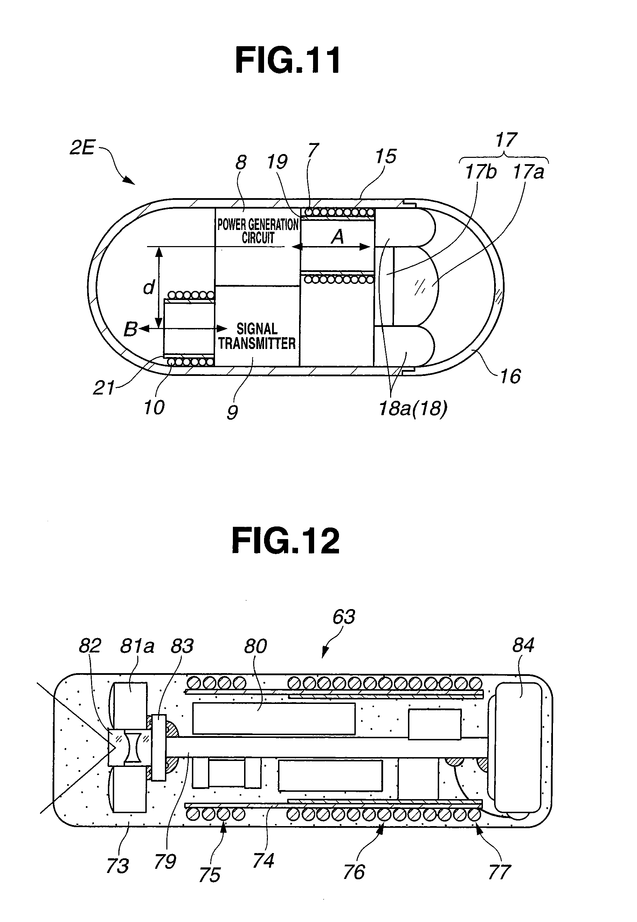

For example, as a conventional example, Japanese Patent Application Laid-Open Publication No. 2005-130943 discloses a capsule-type medical device 63 that is shown in Fig. 12. The capsule-type medical device 63 is formed in a capsule shape by an integrally molded resin 73, and contains therein a transmission coil 75 as a transmission antenna that wirelessly transmits image information, a power receiving coil 76 as a power receiving antenna that receives an AC magnetic field generated by a power transmission antenna from outside a body and generates an AC power, and the like. The transmission coil 75 and the power receiving coil 76 are provided in an embedded state in the capsule-type medical device 63.

More specifically, a coil member 77 that forms the transmission coil 75 used for transmission and the power receiving coil 76 that receives an AC power from outside a body is formed by winding a conductor wire around a cylindrical outer circumferential face of a cylindrical coil core 74. The coil member 77 is arranged inside the integrally molded resin 73.

A plate-shaped substrate 79 is arranged along an axis of the coil core 74. An electronic component 80 is mounted on the substrate 79 to form a circuit that has predetermined functions.

At one end portion of the substrate 79 is arranged an LED substrate 81 a to which is mounted an LED for performing illumination. An objective optical system 82 that forms an optical image is installed in a penetrating hole portion provided in the center of the LED. An image pickup device 83 is arranged at an image-forming position of the objective optical system 82, thereby integrating illumination means and image pickup means.

A capacitor 84 that has a function as electric storage means is arranged at another end portion of the substrate 79. The capacitor 84 is electrically connected to the substrate 79.

According to this conventional example, the transmission coil 75 and the power receiving coil 76 are formed of the same coil member 77.

However, there is the following problem with the above described conventional example.

According to the above described conventional example, because the transmission coil 75 and the power receiving coil 76 are formed of the same coil member 77, the respective coils (antennas) thereof are arranged on the same winding axis.

Consequently, the orientation directions of the respective antennas are on the same straight line. Thus, the antenna of the transmission coil 75 and the power receiving coil 76 as another antenna that has directivity interfere with each other. In particular, there is a risk of increasing noise of a transmission signal that is transmitted from the transmission coil 75.

The present invention has been made in view of the above described circumstances, and an object of the invention is to provide a wireless-type subject in-vivo information acquiring apparatus that, among a transmission antenna and another antenna, can reduce an influence on at least a transmission operation by the transmission antenna.

Another object of the present invention is to provide a wireless-type subject in-vivo information acquiring apparatus that, among a transmission antenna and another antenna, can reduce an influence on at least a transmission operation by the transmission antenna, and can reduce an influence exerted as noise on a transmission signal that is transmitted from the transmission antenna.

### Disclosure of Invention

### Means for Solving the Problem

According to the present invention, there is provided a wireless-type subject in-vivo information acquiring apparatus that is introduced into a body cavity of an individual to be examined and acquires subject in-vivo information, and that has a transmission antenna that emits a transmission signal that transmits acquired subject in-vivo information most strongly in an antenna orientation direction and another antenna that has an antenna directivity in a predetermined direction that is different from that of the transmission antenna,
wherein the transmission antenna and the other antenna are arranged so that a twisted positional relationship or an intersecting relationship exists between the antenna orientation direction of the transmission antenna and an antenna orientation direction of the other antenna.

Further, according to another aspect of the present invention, there is provided a wireless-type subject in-vivo information acquiring apparatus that is introduced into a body cavity of an individual to be examined and acquires subject in-vivo information, and that has a transmission antenna that emits a transmission signal that transmits acquired subject in-vivo information most strongly in an antenna orientation direction and another antenna that has an antenna directivity in a predetermined direction that is different from that of the transmission antenna,
wherein the transmission antenna and the other antenna are arranged so that the antenna orientation direction of the transmission antenna and an antenna orientation direction of the other antenna are parallel, excluding a case in which antenna orientation direction of the transmission antenna and the antenna orientation direction of the other antenna are collinear.

### Brief Description of the Drawings

Fig. 1 is a block diagram that shows the overall configuration of a wireless-type subject in-vivo information acquiring system including a first embodiment of the present invention;
Fig. 2 is a view that illustrates an outline configuration of a wireless-type subject in-vivo information acquiring system by means of a usage example;
Fig. 3 is a view that illustrates a configuration of a wireless-type subject in-vivo information acquiring apparatus according to the first embodiment of the present invention;
Fig. 4 is a circuit diagram that illustrates a configuration of a power generation circuit;
Fig. 5 is an explanatory drawing that illustrates a relationship between orientation directions according to the arrangement of a transmission antenna and a power receiving antenna in a wireless-type subject in-vivo information acquiring apparatus;
Fig. 6 is a view that illustrates a configuration of a wireless-type subject in-vivo information acquiring apparatus according to a modification example;
Fig. 7 is an explanatory drawing that illustrates a relationship between orientation directions according to the arrangement of a transmission antenna and a power receiving antenna in the modification example;
Fig. 8 is a view that illustrates a configuration of a wireless-type subject in-vivo information acquiring apparatus according to a second embodiment of the present invention;
Fig. 9A is a plan view of a planar antenna;
Fig. 9B is a perspective view that illustrates the configuration of the planar antenna;
Fig. 10 is a view that illustrates a configuration of a wireless-type subject in-vivo information acquiring apparatus according to a third embodiment of the present invention;
Fig. 11 is a view that illustrates a configuration of a wireless-type subject in-vivo information acquiring apparatus in which a transmission antenna and another antenna are arranged in parallel; and
Fig. 12 is a view that illustrates a configuration of a capsule-type medical device according to a conventional example.

### Best Mode for Carrying Out the Invention

Embodiments of the present invention are described hereunder with reference to the drawings.

### (First Embodiment)

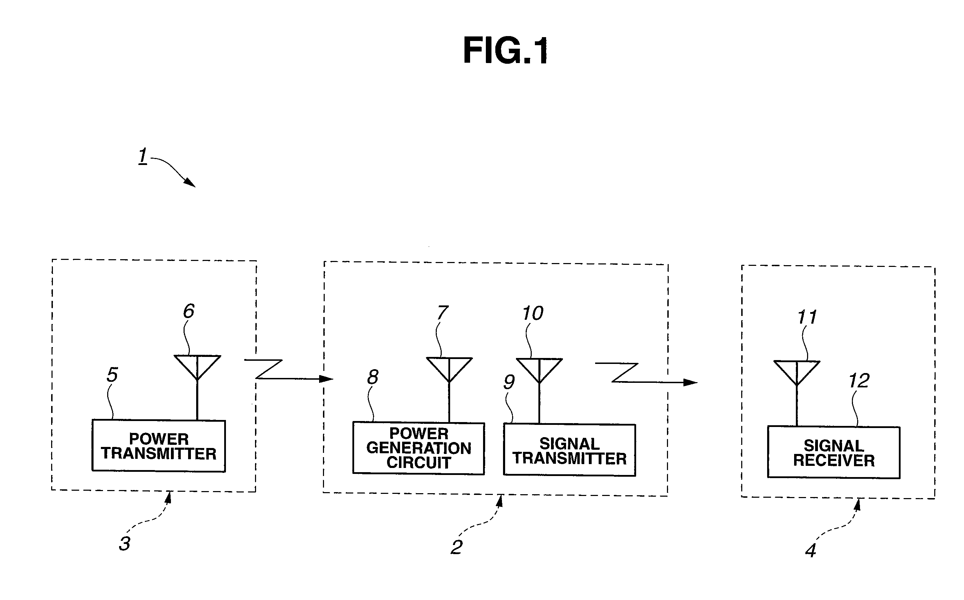

As shown in Fig. 1, a wireless-type subject in-vivo information acquiring system 1 included in a first embodiment of the present invention includes a wireless-type subject in-vivo information acquiring apparatus (hereunder, abbreviated to "subject in-vivo information acquiring apparatus") 2 that is introduced into a body cavity of an individual to be examined and acquires subject in-vivo information, an electric power supply apparatus 3 that wirelessly supplies an electric power to the subject in-vivo information acquiring apparatus 2 from outside the body of the individual to be examined, and a signal receiving apparatus 4 that receives subject in-vivo information that is acquired and wirelessly transmitted by the subject in-vivo information acquiring apparatus 2. In this connection, Fig. 1 shows the configuration of principal parts of each apparatus.

The electric power supply apparatus 3 has an unshown power source, a power transmitter 5 that generates an AC power based on the power source, and a power transmission antenna 6 that transmits (or emits) an AC power that is supplied from the power transmitter 5 as an AC magnetic field or an electric wave.

The subject in-vivo information acquiring apparatus 2 has a power receiving antenna 7 that receives an AC magnetic field generated by the power transmission antenna 6 or an electric wave emitted therefrom and induces an AC power, and a power generation circuit 8 that generates a DC power based on the AC power induced by the power receiving antenna 7.

The subject in-vivo information acquiring apparatus 2 also has a signal transmitter 9 that generates a transmission signal that wirelessly transmits acquired subject in-vivo information, and a transmission antenna 10 that transmits the transmission signal. The signal receiving apparatus 4 has a reception antenna 11 that receives a transmission signal transmitted by the transmission antenna 10, and a signal receiver 12 that reproduces subject in-vivo information from a transmission signal received by the reception antenna 11.

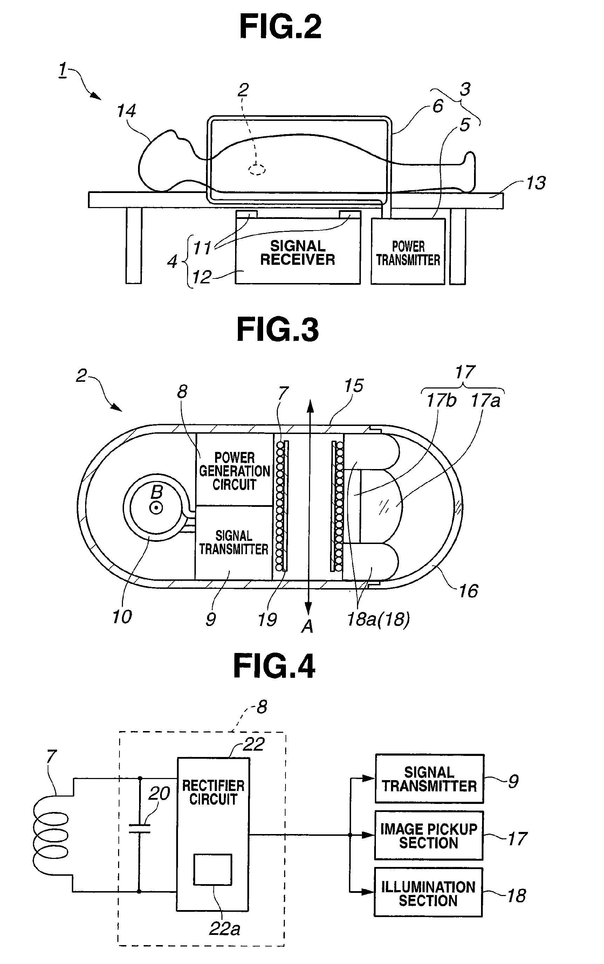

Fig. 2 illustrates the overall configuration of the wireless-type subject in-vivo information acquiring system 1 by means of a usage example. As shown in Fig. 2, an individual to be examined 14 lies down on a bed 13. The subject in-vivo information acquiring apparatus 2 has been introduced into a body cavity of the individual to be examined 14.

The power transmission antenna 6 included in the electric power supply apparatus 3 is mounted at side portions on two sides (only one side is shown in Fig. 2) of the bed 13 such that two sides of the power transmission antenna 6 face each other. The power transmission antenna 6 is connected to a power transmission output end of the power transmitter 5.

An AC magnetic field is generated between the facing sides of the power transmission antenna 6 by an AC power that is supplied from the power transmitter 5. In this connection, the size of the power transmission antenna 6 is set so as to cover an area in which the subject in-vivo information acquiring apparatus 2 that is introduced into the body cavity of the individual to be examined 14 moves within the body cavity.

The signal receiver 12 is arranged on a bottom side of the bed 13. For example, the reception antenna 11 is mounted on a top surface of the signal receiver 12, and the signal receiver 12 receives a transmission signal that is transmitted from the subject in-vivo information acquiring apparatus 2. In the example shown in Fig. 2, a plurality of the reception antennas 11 are provided, and the signal receiver 12 selects the reception antenna 11 that receives the strongest signal and receives the transmission signal from the selected reception antenna 11.

Fig. 3 illustrates a configuration example of the subject in-vivo information acquiring apparatus 2. In the subject in-vivo information acquiring apparatus 2, the power receiving antenna 7 that is formed of a solenoid-shaped coil and the power generation circuit 8, as well as the signal transmitter 9 and the transmission antenna 10 that is formed of a solenoid-shaped coil are housed inside a cylindrical capsule-type container 15 whose two ends are formed in a hemispherical shape.

A transparent cover 16 covers one of the hemispherical-shaped portions of the capsule-type container 15, and an objective lens 17a and an image pickup device 17b that constitute an image pickup section 17 arranged at a central portion on an inner side thereof. The image pickup device 17b is a charge coupled device (abbreviated as "CCD") or a CMOS imager or the like that picks up an image that is disposed at an image-forming position.

A plurality of LEDs 18a, for example, that form an illumination section 18 are arranged around the image pickup section 17. An illuminating light emitted from the plurality of LEDs 18a illuminates the range of field of view (image pickup range) in which an image is to be picked up by the image pickup section 17.

An image pickup signal that is picked up inside a body cavity by the image pickup section 17 is modulated into a transmission signal by the signal transmitter 9, and thereafter is transmitted from the transmission antenna 10.

Although a specific example of the subject in-vivo information acquiring apparatus 2 according to the present embodiment is exemplified in this case by a capsule-type endoscope including the image pickup section 17 that acquires optical information inside a body cavity, for example, the apparatus may include a component that acquires other biological information inside a body cavity such as a pH sensor instead of the image pickup section 17.

The power receiving antenna 7 formed of a solenoid-shaped coil is formed by winding the coil around a cylindrical coil frame 19. Further, the transmission antenna 10 formed of a solenoid-shaped coil is formed by winding the coil around an unshown coil frame. Note that the power receiving antenna 7 and transmission antenna 10 may also be formed without using a coil frame.

Fig. 4 shows a configuration of a power generation circuit 8 provided inside the subject in-vivo information acquiring apparatus 2.

A capacitor 20 is connected to both ends of the power receiving antenna 7 to form a resonant circuit that resonates at a frequency of an AC power that is supplied from the electric power supply apparatus 3. An AC power induced by the resonant circuit is rectified by a rectifier circuit 22 and converted into a DC power that is stored in a power storage section 22a such as a storage capacitor inside the rectifier circuit 22. The DC power that is stored in the power storage section 22a is supplied to the signal transmitter 9, the image pickup section 17, and the illumination section 18 of the subject in-vivo information acquiring apparatus 2 and utilized as a power supply for operation thereof.

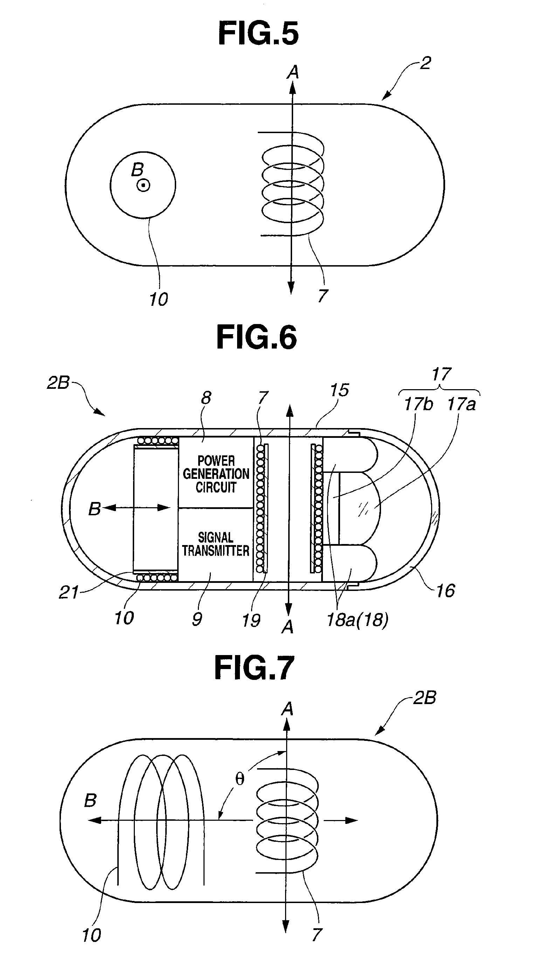

Fig. 5 illustrates the arrangement relationship and the orientation directions with respect to the power receiving antenna 7 and the transmission antenna 10 of the subject in-vivo information acquiring apparatus 2 shown in Fig. 3.

At the power receiving antenna 7, a direction of a central axis of the solenoid-shaped coil is an orientation direction (also referred to as "antenna orientation direction") A in which an induced electric power is most efficiently generated by means of an AC magnetic field. Further, at the transmission antenna 10 also, a direction of a central axis of the solenoid-shaped coil is an orientation direction B in which a transmission signal is most strongly emitted. In this connection, in Fig. 3 and Fig. 5, the orientation direction B is a direction that is perpendicular to the page surface.

According to the present embodiment, with respect to the power receiving antenna 7, for example, a direction that is orthogonal to a cylindrical central axis of the capsule-type container 15 is the orientation direction A. Further, the orientation direction B of the transmission antenna 10 is set in a twisted positional relationship with respect to the orientation direction A in which the orientation direction B is not on the same positional plane as the orientation direction A.

In the case of this twisted positional relationship, the two orientation directions A and B do not intersect.

Note that, in the present description, an angle that the two orientation directions A and B that are in a twisted positional relationship form is defined as an angle at which the two orientation directions meet when an orientation direction of one of the antennas is projected onto a plane that includes an orientation direction of the other antenna.

In the case of the arrangement example shown in Fig. 3 (more specifically, Fig. 5), the orientation direction B is set so that an angle when the orientation direction B is projected onto a plane including the orientation direction A of the power receiving antenna 7 is 90°, that is, a right angle.

Further, according to the present embodiment, the power receiving antenna 7 and the transmission antenna 10 are arranged in a separated manner inside the capsule-type container 15.

Operations of the present embodiment that has the above described configuration will now be described. The power of the electric power supply apparatus 3 and the signal receiving apparatus 4 is turned on and, as shown in Fig. 2, the individual to be examined 14 swallows the subject in-vivo information acquiring apparatus 2 and lies on the bed 13.

Thereupon, the power transmitter 5 supplies an AC power to the power transmission antenna 6 whose sides are facing each other on both sides of the individual to be examined 14 on the bed 13, to thereby generate an AC magnetic field.

An AC power is induced by the AC magnetic field at the power receiving antenna 7 arranged inside the subject in-vivo information acquiring apparatus 2 that has been introduced into a body cavity of the individual to be examined 14. The AC power is converted to a DC power by the rectifier circuit 22 in the power generation circuit 8, and is stored by the power storage section 22a in the rectifier circuit 22.

Subsequently, the illumination section 18, the image pickup section 17, and the signal transmitter 9 to which the DC power is supplied enter an operating state. The image pickup section 17 picks up an image within the body cavity under the illumination of light produced by the illumination section 18. A signal of a picked-up image that has been picked up in this manner is transmitted as a transmission signal from the transmission antenna 10 by the signal transmitter 9.

According to the present embodiment, as shown in Fig. 3 and Fig. 5, the transmission antenna 10 and the power receiving antenna 7 are arranged so as to be in a twisted positional relationship. Further, since an angle formed by the two orientation directions A and B in the twisted positional relationship is set to 90°, it is possible to adequately reduce an effect (adverse effect) whereby the transmission antenna 10 and the power receiving antenna 7 interfere with each other's operation. In particular, when the transmission antenna 10 transmits a transmission signal, since the orientation direction B that transmits the transmission signal most strongly is set so as to be a direction that is orthogonal (and parallel) to the orientation direction A of the power receiving antenna 7, the occurrence of a situation in which an AC power induced by the power receiving antenna 7 affects the transmission antenna 10 as noise can be adequately reduced.

Therefore, according to the present embodiment, with respect to operations performed by the transmission antenna 10 and another antenna, it is possible to reduce an influence on at least a transmission operation performed by the transmission antenna 10. Further, it is possible to adequately reduce the occurrence of a situation in which the power receiving antenna 7 as the other antenna exerts an adverse effect in the form of noise on a transmission signal that is transmitted from the transmission antenna 10.

In the first embodiment as described above, when an angle formed by the two orientation directions A and B that are in a twisted positional relationship is represented by θ, the angle θ is taken to be 90°. However, the present invention is not limited to this angle, and even in the case of an arbitrary angle that satisfies a condition θ° ≤ θ ≤ 90°, the effect is adequately obtained in comparison to the conventional example.

In this connection, the angle θ is defined as being on an acute angle side that includes 90° with respect to an angle formed by two antennas.

The arrangement relationship between the transmission antenna 10 and the power receiving antenna 7 in Fig. 3 as described above may be reversed.

Further, a configuration as shown in Fig. 6 may be adopted as a modification example of the above described embodiment. Relative to the subject in-vivo information acquiring apparatus 2 shown in Fig. 3, in a subject in-vivo information acquiring apparatus 2B shown in Fig. 6 the orientation direction B of the transmission antenna 10 is set so as to be the direction of the central axis of the capsule-type container 15.

That is, the transmission antenna 10 that is formed of a solenoid-shaped coil is wound around an outer circumferential face of a ring-shaped coil frame 21 that is concentric with the central axis of the capsule-type container 15, and is housed inside the capsule-type container 15. Note that the transmission antenna 10 may also be formed without using a coil frame.

Fig. 7 illustrates the arrangement relationship between the power receiving antenna 7 and the transmission antenna 10 of the subject in-vivo information acquiring apparatus 2B as well as the relationship between the orientation directions thereof in this case.

As shown in Fig. 7, the orientation direction A of the power receiving antenna 7 and the orientation direction B of the transmission antenna 10 are orthogonal to each other, and the two orientation directions A and B are included in the same plane. In this case, when an angle at which the two orientation directions A and B intersect is represented by θ, θ = 90°.

In the case of the present modification example, since the two orientation directions A and B are orthogonal and intersect with each other, it is possible to adequately reduce interference between the transmission antenna 10 and the other antenna, similarly to the first embodiment. In particular, it is possible to adequately reduce the occurrence of a situation in which the power receiving antenna 7 as the other antenna influences a transmission operation performed by the transmission antenna 10. Furthermore, the present modification example can adequately reduce an influence that the power receiving antenna 7 exerts in the form of noise on a transmission signal transmitted from the transmission antenna 10.

In this connection, a configuration may also be adopted in which the arrangement relationship between the transmission antenna 10 and the power receiving antenna 7 shown in Fig. 6 is reversed.

Although Fig. 6 illustrates an example in which an angle at which the two orientation directions A and B intersect is an orthogonal angle, the present invention is not limited thereto. For example, even in a case where the two orientation directions A and B intersect at an arbitrary angle that satisfies a condition 30° ≤ θ ≤ 90°, the effect is adequately obtained in comparison to the conventional example.

In this connection, in this case also, an intersecting angle is defmed as an angle in the case of measuring an acute angle including 90°.

### (Second Embodiment)

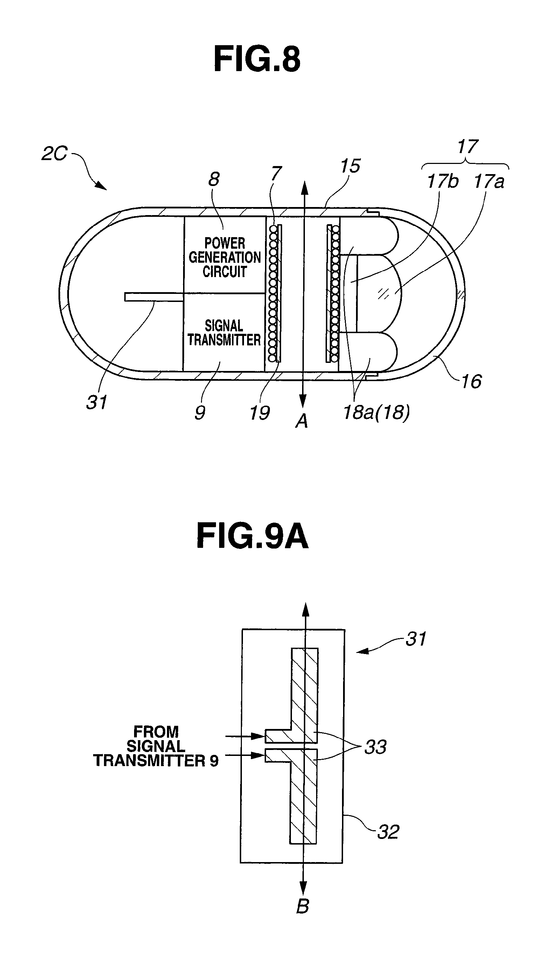

Next, a second embodiment of the present invention is described with reference to Fig. 8 to Fig. 9B. Fig. 8 illustrates a subject in-vivo information acquiring apparatus 2C according to the second embodiment of the present invention. In the subject in-vivo information acquiring apparatus 2C, for example, relative to the subject in-vivo information acquiring apparatus 2 shown in Fig. 3, the transmission antenna 10 is formed using a planar antenna 31 as a planar, conductive pattern antenna. The orientation direction B of the planar antenna 31 shown in Fig. 8 is a direction that is perpendicular to the page surface, similarly to the configuration illustrated in Fig. 3.

As shown in Fig. 9A, in the planar antenna 31, a longitudinal direction of an electric conductor pattern 33 formed on a dielectric planar substrate 32 is the orientation direction B. In this connection, a transmission signal from the signal transmitter 9 is inputted to two end portions formed in an L-shape in the center of the planar antenna 31. The remaining configuration is the same as the configuration illustrated in Fig. 3.

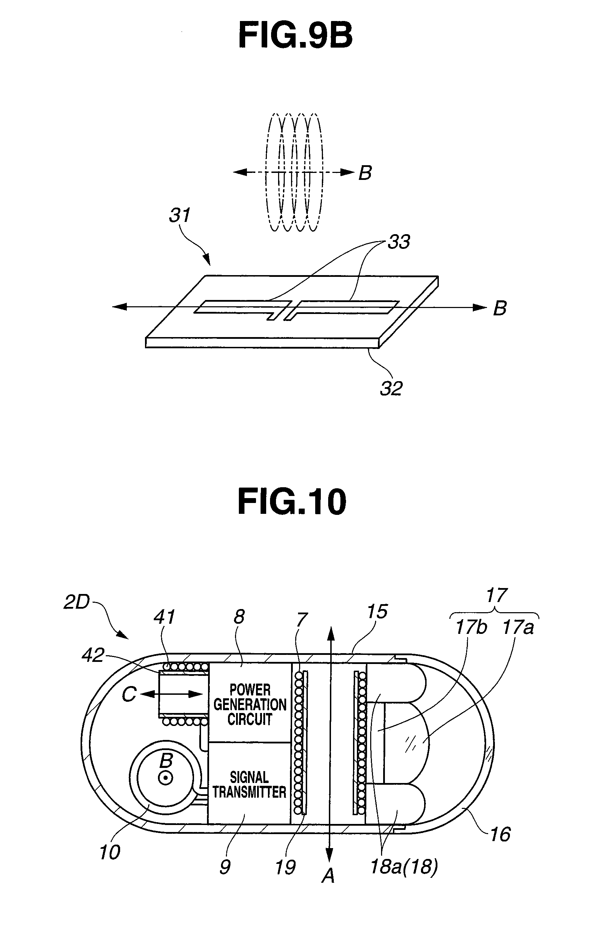

Fig. 9B illustrates the structure of the planar antenna 31 with a perspective view, and also shows the orientation direction B thereof. In the present embodiment, a twisted positional relationship exists between the orientation direction B of the planar antenna 31 and the orientation direction A of the power receiving antenna 7, and an angle θ formed by the orientation direction B and the direction A is 90°. Hence, similarly to the first embodiment, interference between the transmission antenna 10 and the other antenna can be reduced. Further, the noise of a transmission signal that is transmitted from the transmission signal can be reduced.

In the configuration example shown in Fig. 8, a twisted positional relationship exists between the orientation direction B of the planar antenna 31 and the orientation direction A of the power receiving antenna 7, and an angle θ formed by the orientation direction B and the direction A is 90°. However, the present invention is not limited thereto, and for example a configuration may be adopted in which, as an intersecting arrangement, the orientation direction B of the transmission antenna 10 is set as shown in Fig. 6.

### (Third Embodiment)

Next, a third embodiment of the present invention is described referring to Fig. 10. Fig. 10 shows a subject in-vivo information acquiring apparatus 2D according to the third embodiment of the present invention. In the subject in-vivo information acquiring apparatus 2D, for example, relative to the subject in-vivo information acquiring apparatus 2 shown in Fig. 3, an antenna for position detection 41 as a third antenna is wound around a coil frame 42 and arranged adjacent to the transmission antenna 10. Note that the antenna for position detection 41 may also be formed without using a coil frame.

An orientation direction C of the antenna for position detection 41 is, for example, a direction parallel to the longitudinal axis of the capsule-type container 15 that is orthogonal to the orientation direction B of the transmission antenna 10. Further, the orientation direction C is orthogonal to the orientation direction A of the power receiving antenna 7.

The above described antenna for position detection 41 is connected to the signal transmitter 9. A signal of a frequency that is different from a frequency of the transmission signal from the signal transmitter 9, more specifically, a signal for position detection, is supplied to the antenna for position detection 41. The antenna for position detection 41 transmits the signal for position detection.

In this case, the signal receiving apparatus 4 described according to the first embodiment further includes a function for receiving the signal for position detection.

The position of the antenna for position detection 41, that is, the position of the subject in-vivo information acquiring apparatus 2 is detected by receiving the signal for position detection together with the transmission signal. The position information is sent to the electric power supply apparatus 3. The electric power supply apparatus 3 utilizes the position information for control that moves the position of the power transmission antenna 6. In this case, by performing control that moves the position of the power transmission antenna 6 in accordance with the position of the subject in-vivo information acquiring apparatus 2, even if the size of the power transmission antenna 6 is small it is possible to supply an amount of AC power required for operation of the subject in-vivo information acquiring apparatus 2 to the power receiving antenna 7 thereof.

According to the present embodiment also, since the orientation direction B of the transmission antenna 10 and the orientation direction C of the antenna for position detection 41 are in an orthogonal intersecting relationship, interference between the two antennas 10 and 41 can be adequately reduced.

Accordingly, an effect whereby the antenna for position detection 41 exerts an influence on a transmission signal in the form of noise can be adequately reduced. Further, a similar effect as in the first embodiment can be achieved with respect to the transmission antenna 10 and the power receiving antenna 7.

Further, with respect to the antenna for position detection 41 and the power receiving antenna 7, the present embodiment has an effect that can adequately reduce interference between the two antennas 41 and 7 in a similar manner to the case of the transmission antenna 10 and the power receiving antenna 7.

Note that, as a modification example of the present embodiment, a configuration may be adopted so that a twisted positional relationship is formed with respect to the orientation direction B of the transmission antenna 10 and the orientation direction C of the antenna for position detection 41.

Further, an angle θ formed by a twist or an intersecting angle θ as described above are not limited to the case of the arrangement example illustrated in Fig. 10.

In this connection, according to the conventional example shown in Fig. 12 as described above, because the orientation directions of two antennas are matching, interference is liable to occur between the two antennas. In contrast, according to the present embodiment, even if the orientation directions of two antennas are parallel, when a configuration is adopted so as to form a distance d between the orientation directions A and B of the two antennas 7 and 10 as shown in Fig. 11, interference between the two antennas 7 and 10 can be reduced in comparison to the conventional example shown in Fig. 12.

In this connection, the arrangement example of the two antennas 7 and 10 shown in Fig. 11 shows a more preferable arrangement example for a case where at least the orientation directions A and B of the two antennas 7 and 10 do not match (that is, d > 0).

In a subject in-vivo information acquiring apparatus 2E shown in Fig. 11, for example, relative to the subject in-vivo information acquiring apparatus 2B shown in Fig. 6, the size of the solenoid-shaped coil of the transmission antenna 10 is reduced and the transmission antenna 10 is arranged on a lower side of the capsule-type container 15. Further, according to the configuration shown in Fig. 11, relative to the configuration shown in Fig. 6, the direction in which the power receiving antenna 7 is arranged is changed so that the power receiving antenna 7 is arranged in parallel with the orientation direction A of the transmission antenna 10.

Further, according to the configuration shown in Fig. 11, the positions at which the two antennas 7 and 10 are arranged are also such that the two antennas 7 and 10 are arranged in a separated manner in the longitudinal axis direction of the capsule-type container 15. Thus, interference between the two antennas 7 and 10 is reduced.

With respect to the aforementioned distance d, preferably, an overlapping portion of the cross-sectional areas of the respective coils that characterize the orientation directions (constitute the respective antennas) is small. Further, as shown in Fig. 11, if the distance d is set so as to be greater than or equal to the sum of the radii of both coils, interference between the two antennas 7 and 10 can be reduced and a more preferable configuration can be realized.

Although a case is illustrated in Fig. 11 in which the orientation directions A and B of the two antennas 7 and 10 are set in a direction that is parallel to the longitudinal axis of the capsule-type container 15, a configuration may be adopted in which the orientation directions A and B are orthogonal to the longitudinal axis of the capsule-type container 15.

Note that, in addition to the power receiving antenna 7 and the antenna for position detection 41 that are described above, an antenna for detecting a direction of the subject in-vivo information acquiring apparatus or an antenna for receiving a command that controls a state of the subject in-vivo information acquiring apparatus or the like can also be applied as another antenna that is different from the transmission antennas 10 and 31 according to the present invention.

Note that an embodiment configured by partially combining the above described embodiments and the like also belongs to the present invention.

### Industrial Applicability

The present invention is introduced into the body of an individual to be examined and acquires in-vivo information such as an image that is picked up inside the body.

This application claims priority from Japanese Patent Application No. 2008-284715 filed in Japan on November 5, 2008, the contents of which are hereby incorporated by reference in their entirety into the description of this application and the claims.

## Claims

1. A wireless-type subject in-vivo information acquiring apparatus that is introduced into a body cavity of an individual to be examined and acquires subject in-vivo information, comprising a transmission antenna that emits a transmission signal that transmits acquired subject in-vivo information most strongly in an antenna orientation direction and another antenna that has an antenna directivity in a predetermined direction that is different from that of the transmission antenna,
wherein the transmission antenna and the other antenna are arranged so that a twisted positional relationship or an intersecting relationship exists between the antenna orientation direction of the transmission antenna and an antenna orientation direction of the other antenna.

2. The wireless-type subject in-vivo information acquiring apparatus according to claim 1, wherein the transmission antenna and the other antenna are arranged so that a relationship between the antenna orientation direction of the transmission antenna and the antenna orientation direction of the other antenna is an orthogonal, twisted positional relationship or a relationship in which the antenna orientation direction of the transmission antenna and the antenna orientation direction of the other antenna intersect at right angles.

3. The wireless-type subject in-vivo information acquiring apparatus according to claim 1, wherein the transmission antenna and the other antenna are configured using at least one of a solenoid-shaped coil in which a direction of a central axis is the antenna orientation direction, and a planar antenna in which a planar, electric conductor pattern is formed in which a direction in which a conductive pattern that is formed on a flat surface extends is the antenna orientation direction.

4. The wireless-type subject in-vivo information acquiring apparatus according to claim 2, wherein the transmission antenna and the other antenna are configured using at least one of a solenoid-shaped coil in which a direction of a central axis is the antenna orientation direction and a planar antenna in which a planar, electric conductor pattern is formed in which a direction in which a conductive pattern that is formed on a flat surface extends is the antenna orientation direction.

5. The wireless-type subject in-vivo information acquiring apparatus according to claim 1, wherein: the other antenna comprises a first antenna and a second antenna that is different from the first antenna; and the transmission antenna, the first antenna, and the second antenna are arranged so that a relationship between the respective antenna orientation directions thereof and other antenna orientation directions is a twisted positional relationship or an intersecting relationship.

6. The wireless-type subject in-vivo information acquiring apparatus according to claim 2, wherein the other antenna comprises a first antenna and a second antenna that is different from the first antenna; and the transmission antenna, the first antenna, and the second antenna are arranged so that a relationship between the respective antenna orientation directions thereof and other antenna orientation directions is an orthogonal, twisted positional relationship or a relationship in which the antenna orientation directions intersect at right angles.

7. The wireless-type subject in-vivo information acquiring apparatus according to claim 1, wherein the other antenna has a power receiving antenna for generating an AC power, and the power receiving antenna is connected to a power generation circuit that generates a DC power.

8. The wireless-type subject in-vivo information acquiring apparatus according to claim 2, wherein the other antenna has a power receiving antenna for generating an AC power, and the power receiving antenna is connected to a power generation circuit that generates a DC power.

9. The wireless-type subject in-vivo information acquiring apparatus according to claim 6, wherein the first antenna is a power receiving antenna for generating an AC power, and the power receiving antenna is connected to a power generation circuit that generates a DC power.

10. The wireless-type subject in-vivo information acquiring apparatus according to claim 8, wherein the power receiving antenna is configured by a solenoid-shaped coil.

11. The wireless-type subject in-vivo information acquiring apparatus according to claim 9, wherein the power receiving antenna is configured by a solenoid-shaped coil.

12. The wireless-type subject in-vivo information acquiring apparatus according to claim 11, further comprising an image pickup section that picks up an image inside a subject, and a transmission circuit that generates a transmission signal that wirelessly transmits an image pickup signal that is picked up by the image pickup section.

13. The wireless-type subject in-vivo information acquiring apparatus according to claim 2, wherein the transmission antenna and the other antenna are housed inside a cylindrical capsule-type container, and the antenna orientation direction of at least one of the transmission antenna and the other antenna is set in a direction that is orthogonal to a center axis direction of a circular cylinder of the capsule-type container.

14. The wireless-type subject in-vivo information acquiring apparatus according to claim 2, wherein the transmission antenna and the other antenna are housed inside a cylindrical capsule-type container, and the antenna orientation direction of at least one of the transmission antenna and the other antenna is set in a center axis direction of a circular cylinder of the capsule-type container.

15. The wireless-type subject in-vivo information acquiring apparatus according to claim 6, wherein the second antenna is an antenna that transmits a transmission signal to outside of the wireless-type subject in-vivo information acquiring apparatus at a frequency that is different from a frequency of the transmission antenna.

16. The wireless-type subject in-vivo information acquiring apparatus according to claim 9, wherein the second antenna is an antenna that transmits a second transmission signal at a frequency that is different from a frequency of the transmission antenna to an electric power supply apparatus that is outside the wireless-type subject in-vivo information acquiring apparatus and that wirelessly supplies an electric power to the power receiving antenna, and the electric power supply apparatus detects a position of the antenna based on the second transmission signal.

17. The wireless-type subject in-vivo information acquiring apparatus according to claim 9, wherein the second antenna is an antenna that transmits a second transmission signal at a frequency that is different from a frequency of the transmission antenna to an electric power supply apparatus that is outside the wireless-type subject in-vivo information acquiring apparatus and that wirelessly supplies an electric power to the power receiving antenna, and the electric power supply apparatus is utilized in control of a power transmission antenna that wirelessly supplies an electric power to the power receiving antenna based on the second transmission signal.

18. The wireless-type subject in-vivo information acquiring apparatus according to claim 9, wherein the second antenna is an antenna that transmits a second transmission signal at a frequency that is different from a frequency of the transmission antenna to an electric power supply apparatus that is outside the wireless-type subject in-vivo information acquiring apparatus and that wirelessly supplies an electric power to the power receiving antenna, and the electric power supply apparatus is utilized for movement of a position of a power transmission antenna that wirelessly supplies an electric power to the power receiving antenna based on the second transmission signal.

19. A wireless-type subject in-vivo information acquiring apparatus that is introduced into a body cavity of an individual to be examined and acquires subject in-vivo information, comprising a transmission antenna that emits a transmission signal that transmits acquired subject in-vivo information most strongly in an antenna orientation direction and another antenna that has an antenna directivity in a predetermined direction that is different from that of the transmission antenna,
wherein the transmission antenna and the other antenna are arranged so that the antenna orientation direction of the transmission antenna and an antenna orientation direction of the other antenna are parallel, excluding a case in which antenna orientation direction of the transmission antenna and the antenna orientation direction of the other antenna are collinear.

20. The wireless-type subject in-vivo information acquiring apparatus according to claim 19, wherein the transmission antenna and the other antenna are formed using a solenoid-shaped first coil and a solenoid-shaped second coil, respectively, and are arranged so that a distance between a central axis of the first coil and a central axis of the second coil is greater than or equal to a sum of a radius of the first coil and a radius of the second coil.
